# EUROPÄISCHE PATENTSCHRIFT

(11) **EP 3 928 680 B1**
(45) Veröffentlichungstag und Bekanntmachung des Hinweises auf die Patenterteilung: **28.09.2022**
(21) Anmeldenummer: 21178951.6
(22) Anmeldetag: 11.06.2021
(51) Int. Cl.: A61B 1/00

(54) **VERFAHREN ZUR ENDOSKOPISCHEN BILDGEBUNG, ENDOSKOPISCHES BILDGEBUNGSSYSTEM UND SOFTWAREPROGRAMMPRODUKT**
ENDOSCOPIC IMAGING METHOD, ENDOSCOPE IMAGING SYSTEM, AND SOFTWARE PROGRAM PRODUCT
PROCÉDÉ D'IMAGERIE ENDOSCOPIQUE, SYSTÈME D'IMAGERIE ENDOSCOPIQUE ET PRODUIT DE PROGRAMME LOGICIEL

(30) Priorität: 23.06.2020 DE 102020116473
(43) Veröffentlichungstag der Anmeldung: 29.12.2021
(73) Patentinhaber: OLYMPUS Winter & Ibe GmbH, 22045 Hamburg (DE)
(72) Erfinder: JÜRGENS, Thorsten, 21037 Hamburg (DE)
(74) Vertreter: Seemann & Partner Patentanwälte mbB

(56) Entgegenhaltungen:
- DE-B3-102011 123 037
- US-A1- 2011 295 062
- US-A1- 2016 120 398
- US-A1- 2020 138 265

## Beschreibung

Die Erfindung betrifft ein Verfahren zur endoskopischen Bildgebung, ein endoskopisches Bildgebungssystem und ein Softwareprogrammprodukt.

Moderne endoskopische Bildgebungssysteme bieten eine Vielzahl von Speziallicht-Bildgebungsmodi neben dem für das visuelle Spektrum (VIS) ausschlaggebenden Weißlicht-Modus unter Weißlicht-Beleuchtung, welche das volle RGB-Spektrum umfasst. Solche Speziallicht-Bildgebungsmodi (engl. *"Special Light Imaging",* SLI) sind beispielsweise die Schmalband-Bildgebung (engl. *"Narrow Band Imaging",* NBI), Nahinfrarot-Fluoreszenz-Bildgebung (engl. *"Near Infrared Fluorescence Imaging",* NIRF) oder Rot-Dichromatische Bildgebung (engl. *"Red Dichromatic Imaging",* RDI). Bei RDI handelt es sich um einen proprietäres Verfahren von Olympus Technology, welches in den US Patenten US 10,034,600 B2 und US 9,775,497 B2 beschrieben ist. Diese Liste ist nicht abschließend.

Nahinfrarot-Fluoreszenz-Bildgebung (NIRF) kann verwendet werden, um die Durchströmung (Perfusion) von Blutgefäßen zu analysieren und zu bewerten, die Anatomie des hepatobiliären Systems zu bestätigen, Lymphknoten zu finden oder den Harnleiter (Ureter) nach der Gabe eines extrinsischen Kontrastmittels wie ICG (Indocyaningrün), Cy5.5, ZW800 oder ZW-1 zu visualisieren. Rot-Dichromatische Bildgebung (RDI) kann verwendet werden, den Ursprung arterieller Blutungen zu identifizieren. Schmalband-Bildgebung (NBI) kann dabei helfen, zwischen einer gutartigen Hyperplasie und krebsartigen Geweben oder Vorstufen von krebsartigen Geweben zu differenzieren, beispielsweise zwischen Darmpolypen der Typen NICE-1 und NICE-2, um zu entscheiden, ob ein Polyp reseziert werden muss oder nicht.

US 2020/138265 A1 beschreibt ein weiteres endoskopisches Bildgebungssystem.

Typische Untersuchungen und Eingriffe erfolgen durch einen Arzt unter Verwendung einer Weißlicht-Beleuchtung zur visuellen Bildgebung (VIS). Entdeckt der Arzt in den Weißlicht-Bildern eine Auffälligkeit, die eine nähere Untersuchung in einem Speziallicht-Bildgebungsmodus erfordert, so wählt der Arzt den passenden Speziallicht-Bildgebungsmodus aufgrund seiner Ausbildung und seiner Erfahrung aus.

Demgegenüber liegt der vorliegenden Erfindung die Aufgabe zugrunde, den Arzt bei der Untersuchung oder dem Eingriff durch eine verbesserte Art der endoskopischen Bildgebung zu unterstützen. Diese Aufgabe wird durch ein Verfahren zur endoskopischen Bildgebung mit einem endoskopischen Bildgebungssystem gelöst, das ein Videoendoskop mit wenigstens einem CMOS-Bildsensor, eine Lichtquelleneinheit, die zur Erzeugung von Weißlicht sowie von wenigstens einem Speziallicht ausgebildet ist, eine Bildauswertungseinheit, eine Steuereinheit und eine Anzeigevorrichtung umfasst, das dadurch weitergebildet ist, dass mit dem Videoendoskop unter Weißlichtbeleuchtung Weißlichtbilder aufgenommen und von der Bildauswertungseinheit in Echtzeit auf das Vorhandensein wenigstens einer Struktur mit wenigstens einer vordefinierten Charakteristik hin ausgewertet werden, wobei, wenn die Bildauswertungseinheit in wenigstens einem Weißlichtbild das Vorhandensein wenigstens einer Struktur mit wenigstens einer vordefinierten Charakteristik erkennt, ein Speziallicht-Bildgebungsmodus eingestellt wird, in welchem die Lichtquelleneinheit eine Speziallichtbeleuchtung mit dem wenigstens einen Speziallicht erzeugt und eine oder mehrere Bildaufnahmen eines Videostreams unter der Speziallichtbeleuchtung aufgenommen wird oder werden, die einer Bildverarbeitung in einem Speziallichtverarbeitungsmodus unterzogen wird oder werden, welche umfasst, dass ein Teilbereich des Weißlichtbildes identifiziert wird, der die im Weißlichtbild erkannte Struktur mit vordefinierter Charakteristik enthält und nur dieser Teilbereich des Bildsensors ausgelesen wird, wobei die aus dem Teilbereich ausgelesenen Bilddaten als Speziallichtbild oder Speziallichtbilder weiterverarbeitet werden.

Das erfindungsgemäße Verfahren beruht darauf, dass die Mehrheit der endoskopischen Videosysteme, also Videoendoskope oder Kameraköpfe für Endoskope, mit CMOS-Bildsensor bestückt sind, anstelle von CCD-Bildsensoren. Viele CMOS-Bildsensoren können im Unterschied zu CCD-Bildsensoren auch nur in Teilbereichen, sogenannten *"Regions of Interest"* (ROI), ausgelesen werden, was die Auslesegeschwindigkeit stark erhöht. Der Teilbereich ist typischerweise ein rechteckiger Bereich, der im Rahmen der Erfindung so bemessen wird, dass er die Struktur vollständig beinhaltet, gegebenenfalls mit einem Rand, der beispielsweise 2 % bis 20 % der Erstreckung der längeren Seite des Rechtecks einnimmt. Beispielsweise nimmt ein Darmpolyp im Bild typischerweise etwa 10 % bis 20 % der Bildfläche ein. In vielen klinischen Situationen hat eine solche ROI im vollen Bild eine entsprechende Ausdehnung. In einem solchen Fall kann ein CMOS-Bildsensor, der beispielsweise eine HD-Auflösung von 1980 x 1080 Pixeln hat, ausschließlich und sehr schnell in einem entsprechenden Teilbereich ausgelesen werden, der den Polypen enthält, beispielsweise in einem rechteckigen Bereich, dessen gegenüberliegende Eckpunkte beispielsweise die Koordinaten 600/200 und 1061/662 haben. Die resultierende, den Darmpolypen abdeckende ROI hätte dann eine Größe von 461 × 462 Pixeln (ca. 213.000 Pixel) und somit nur etwa ein Zehntel der Größe des vollständigen HD-Bildes.

Mit dem erfindungsgemäßen Verfahren ist es somit möglich, eine hohe Bildwiederholfrequenz auch dann zu erhalten, wenn ein Speziallicht-Bildgebungsmodus eingeschaltet ist, weil das Auslesen und Verarbeiten der Teilbereiche des CMOS-Bildsensors schneller erfolgt als das Auslesen der vollständigen Bilddaten des CMOS-Bildsensors im Weißlicht-Bildgebungsmodus. Ohne die Auswahl von Teilbereichen würde die Bildwiederholfrequenz bei einer alternierenden Folge von Weißlichtbildern und Speziallichtbilder halbiert werden, beispielsweise von 50 Hz auf 25 Hz, wobei die Weißlicht-Bildfolge bei 25 Hz und die Speziallicht-Bildfolge ebenfalls bei 25 Hz läge. Bei schnellen Bewegungen könnte dies zu Bewegungsartefakten führen.

Viele Speziallicht-Bildgebungsmodi sind außerdem sehr lichtschwach. Die erforderliche hohe Lichtempfindlichkeit bzw. Verstärkungsfaktoren führen zu starkem Bildrauschen. Das Rauschen kann auch reduziert werden, indem die Belichtungszeit erhöht wird oderaufeinanderfolgende Frames miteinander kombiniert werden. Diese Maßnahmen sind bei einer normalen zeitsequenziellen Bildgebung ohne Auswahl von Teilbereichen stark eingeschränkt, während die erfindungsgemäß mögliche höhere Bildwiederholungsrate unter Speziallichtbedingungen genutzt werden kann, die effektive Belichtungszeit der Speziallichtbilder über deren dichtere Abfolge zu erhöhen.

Das erfindungsgemäße Verfahren ermöglicht es zudem, den behandelnden Arzt dadurch zu unterstützen, dass eine Bilderkennung an den Weißlichtbildern durchgeführt wird, in der automatisch Strukturen mit vordefinierter Charakteristik erkannt werden. Solche Strukturen können beispielsweise durchströmte Blutgefäße, Lymphknoten, arterielle Blutungen oder Darmpolypen sein, wobei diese Auflistung nicht abschließend ist. Die vordefinierten Charakteristika dieser Strukturen sind aufgrund umfangreichen Bildmaterials gut bekannt und beschrieben und lassen sich in einer automatisierten Bildauswertung identifizieren. Erfindungsgemäß wird in diesem Fall eine geeignete Speziallicht-Bildgebung eingestellt, mit der die gefundene Struktur näher und genauer dargestellt wird. Dem Arzt ist die Entscheidung zur Einstellung des Speziallicht-Bildgebungsmodus abgenommen, und er bekommt ohne eigenes Zutun die Speziallicht-Bildgebung angezeigt.

Eine laufende Untersuchung wird üblicherweise als Videostream verarbeitet. Das erfindungsgemäße Verfahren kann auf den laufenden Videostream angewendet werden, aber auch auf Wunsch des Arztes auf Einzelbilder angewendet werden, die aus dem Videostream durch eine Snapshot-Funktion isoliert worden sind und die zur genaueren Untersuchung und Dokumentation dienen können.

Die Auswahl des Teilbereichs erfolgt in der Bildauswertungseinheit, die Anweisung, welcher Teilbereich des CMOS-Bildsensors ausgelesen wird, erfolgt entweder von der Bildauswertungseinheit direkt oder über die Steuereinheit. Die Bildauswertungseinheit kann auch programmiertechnisch als Funktionseinheit, beispielsweise als Programm oder Subroutine eines Programms in die Steuereinheit integriert sein.

Bei verschiedenen Untersuchungen ist der Normalzustand, dass keine interessierenden Strukturen zu sehen sind. In diesen Fällen werden vorteilhafterweise die Weißlichtbilder mittels der Anzeigevorrichtung angezeigt, wenn die Bildauswertungseinheit in den Weißlichtbildern keine Struktur mit wenigstens einer vordefinierten Charakteristik erkennt.

In Ausführungsformen wird oder werden das Speziallichtbild oder die Speziallichtbilder alleine oder als Kompositbild oder Kompositbilder in einer Überlagerung über Weißlichtbilder mittels der Anzeigevorrichtung angezeigt. Die Anzeige des Speziallichtbildes alleine hat den Vorteil einer ungestörten Darstellung, die für das geübte Auge des Arztes gut zu interpretieren ist. Die Anzeige eines Kompositbildes, bei dem das Speziallichtbild dem Weißlichtbild an der Stelle der Struktur deckungsgleich überlagert ist, bietet den Vorteil der Einbettung des Speziallichtbildes in die umliegenden Strukturen. Ein solches fusioniertes Bild oder Kompositbild kann als *Augmented Reality Image* (ARI) bezeichnet werden. Für das Kompositbild kann das Speziallichtbild mit einer gewissen Transparenz, etwa zwischen 5 % und 50 % über das Weißlichtbild überlagert werden, so dass auch die unterliegenden Strukturen des Weißlichtbildes, die im Speziallichtbild unauffällig sind, weiterhin sichtbar sind, oder intransparent mit einem Transparenzgrad von 0 % bis 5 %.

In Ausführungsformen mit im Videostream angezeigter Kompositbildfolge wird in dem Fall, wenn die Bildauswertungseinheit in den Weißlichtbildern das Vorhandensein wenigstens einer Struktur mit wenigstens einer vordefinierten Charakteristik erkennt, eine mit der Video-Bildfolge synchronisierte alternierende Weißlicht- und Speziallichtbeleuchtung erzeugt, wobei jeweils ein Paar aus Weißlichtbild und Speziallichtbild verarbeitet und zu einem Kompositbild zusammengesetzt wird. Auch aus diesem Kompositbild-Videostream können per Snapshot einzelne Frames des Kompositbild-Stroms festgehalten werden und zur näheren Untersuchung und Dokumentation verwendet werden. Der Begriff des Speziallicht-Bildgebungsmodus umfasst im Zusammenhang mit der vorliegenden Patentanmeldung und der vorliegenden Erfindung daher auch den Fall, dass Weißlichtbilder und Speziallichtbilder in alternierender Folge aufgenommen, weiterverarbeitet und zusammengesetzt werden.

Die Video-Bildfolge und die synchronisierte alternierende Weißlicht- und Speziallichtbeleuchtung erfolgt in einer Ausführungsform in einem zeitlichen Rhythmus, in dem die Dauer der Speziallichtbeleuchtung und Speziallichtbildaufnahme kürzer ist als die Dauer der Weißlichtbeleuchtung und Weißlichtbildaufnahme. Dies ist möglich, da für die Speziallichtbildaufnahmen jeweils nur ein Teilbereich des CMOS-Bildsensors ausgelesen wird und nur die Bilddaten dieses Teilbereichs in der Bildauswertungseinheit weiterverarbeitet werden, zumal auf die Speziallichtbilder keine Strukturerkennung angewandt wird. Somit ist es nicht mehr notwendig, die Frequenz der Bilddarstellung, d. h. der Abfolge der einzelnen Frames, zu halbieren, sondern es kann eine Bildabfolge mit einer ähnlich hohen Frequenz wie die reine Weißlicht-Videoabfolge erreicht werden, wodurch Bewegungsartefakte weitgehend vermieden werden. Eine Möglichkeit, dies zu verwirklichen besteht darin, dass die Steuereinheit, die auch mit der Bildauswertungseinheit integriert sein kann, den Bildsensor im Videoendoskop und die Lichtquelleneinheit anweist, in entsprechend synchronisierter zeitlicher Abfolge Bilder bzw. Teilbilder aufzunehmen einerseits und Weißlichtbeleuchtung und Speziallichtbeleuchtung andererseits zur Verfügung zu stellen.

In Ausführungsformen ist die Lichtquelleneinheit ausgebildet, ein oder mehrere aus einer Beleuchtung für Schmalband-Bildgebung (NBI), einer Beleuchtung für Nahinfrarotfluoreszenz-Bildgebung (NIRF) und einer Beleuchtung für eine Rot-Dichromatische-Bildgebung (RDI) als Speziallicht zu erzeugen, wobei insbesondere bei erkannter Blutung eine RDI-Beleuchtung erzeugt wird und/oder bei erkannten Darmpolypen eine NBI-Beleuchtung erzeugt wird. Die Bildauswertungsvorrichtung kann ausgebildet sein, anhand der erkannten Struktur mit vordefinierter Charakteristik eine aus mehreren zur Verfügung stehenden Speziallicht-Modi auszuwählen. Alternativ kann vorgesehen sein, dass die vordefinierte Charakteristik und der Speziallicht-Modus vorausgewählt und eingestellt ist oder wird. Dies kann beispielsweise bei Untersuchungen nützlich sein, bei denen nach bestimmten Strukturen gesucht wird, beispielsweise bei Darmspiegelungen oder bei Operationen, bei denen Blutungen zu erwarten sind, die aufzuspüren und zu stillen sind.

In Ausführungsformen erfolgt die Erkennung der Struktur mit wenigstens einer vordefinierten Charakteristik und/oder die Auswahl des die Struktur enthaltenden Teilbereichs und/oder die Auswahl des Speziallicht-Modus mittels eines auf künstlicher Intelligenz basierenden Bildauswertungsalgorithmus. Der Bildauswertungsalgorithmus kann in solchen Fällen auf einem oder mehreren klassifizierenden Neuronalen Netzen, insbesondere CNN, beruhen, welches oder welche mit Weißlichtbildern vortrainiert wurde oder wurden, die Strukturen mit wenigstens einer vordefinierten Charakteristik enthalten.

In einer Erweiterung ist in Ausführungsformen vorgesehen, dass die Bildauswertungseinheit die Speziallichtbilder auf das Vorhandensein einer entsprechenden Struktur hin auswertet, insbesondere die Anwesenheit der Struktur bestätigt oder anderenfalls veranlasst, den Speziallicht-Bildgebungsmodus wieder zu verlassen. Die Speziallicht-Bildgebungsmodi sind besonders geeignet, die fraglichen Strukturen sehr kontrastreich und damit gut wiedererkennbar hervortreten zu lassen, so dass eine Auswertung der Speziallichtbilder bezüglich dieser Strukturen eine hohe Signifikanz bezüglich des Vorhandenseins entsprechender Strukturen bringt. Eine entsprechende Bildauswertung ist ebenfalls schneller als bei den Weißlichtbildern, weil dann nur ein Teilbereich des Weißlichtbildes im Speziallichtbild vorhanden ist und somit eine kleinere Datenmenge auszuwerten ist.

Die der Erfindung zugrunde liegende Aufgabe wird auch durch ein endoskopisches Bildgebungssystem gelöst, umfassend ein Videoendoskop mit wenigstens einem CMOS-Bildsensor, eine Lichtquelleneinheit, die zur Erzeugung von Weißlicht sowie von wenigstens einem Speziallicht ausgebildet ist, eine Bildauswertungseinheit, eine Steuereinheit und eine Anzeigevorrichtung, wobei die Steuereinheit mit der Lichtquelleneinheit, dem Videoendoskop und der Bildauswertungseinheit signaltechnisch verbunden ist und die Bildauswertungseinheit mit dem Videoendoskop signaltechnisch verbunden ist, wobei das Bildgebungssystem mit dem Videoendoskop, der Lichtquelleneinheit, der Bildauswertungseinheit, der Steuereinheit und der Anzeigevorrichtung ausgebildet und eingerichtet ist, ein zuvor beschriebenes erfindungsgemäßes Verfahren auszuführen.

Das Videoendoskop des erfindungsgemäßen Bildgebungssystems kann entweder im distalen Bereich des Endoskopschafts oder im Handgriff einen CMOS-Sensor aufweisen oder als Kombination eines Endoskops mit einem Kamerakopf ausgebildet sein, welcher einen CMOS-Sensor als Bildsensor aufweist.

Ferner wird die der Erfindung zugrunde liegende Aufgabe auch durch ein Softwareprogrammprodukt mit Programmcodemitteln für ein erfindungsgemäßes endoskopisches Bildgebungssystem, mit einer Bildauswertungsprogrammkomponente, die in der Bildauswertungseinheit des Bildgebungssystems abläuft, und einer Steuerprogrammkomponente, die in der Steuereinheit Bildgebungssystems abläuft, wobei die Bildauswertungsprogrammkomponente und die Steuerprogrammkomponente ausgebildet sind, ein zuvor beschriebenes erfindungsgemäßes Verfahren auszuführen, wenn sie in der Bildauswertungseinheit und der Steuereinheit ablaufen.

Das erfindungsgemäße endoskopische Bildgebungssystem und das erfindungsgemäße Softwareprogrammprodukt beziehen sich auf das zuvor beschriebene erfindungsgemäße Verfahren und verwirklichen dessen Vorteile, Merkmale und Eigenschaften in gleicher Weise.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Darstellung eines erfindungsgemäßen endoskopischen Bildgebungssystems,
- Fig. 2: eine schematische Darstellung des Bildgebungskonzepts und
- Fig. 3: ein beispielhaftes Ablaufdiagramm für ein erfindungsgemäßes Verfahren.

In den Zeichnungen sind jeweils gleiche oder gleichartige Elemente und/oder Teile mit denselben Bezugsziffern versehen, so dass von einer erneuten Vorstellung jeweils abgesehen wird.

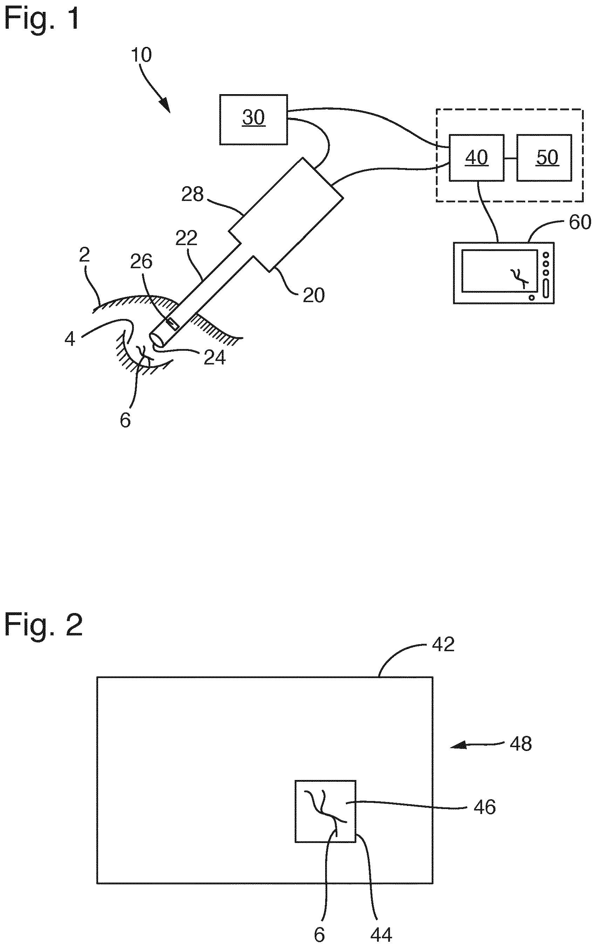

Fig. 1 zeigt eine schematische Darstellung eines erfindungsgemäßen endoskopischen Bildgebungssystems 10 in einer Operationssituation. Mittels eines Videoendoskops 20 wird in einem Körperhohlraum 4 eines Körpers 2 eines Patienten eine Untersuchung durchgeführt, bei der eine Struktur 6 mit einer vordefinierten Charakteristik entdeckt wird, beispielsweise ein Darmpolyp, eine Blutung, ein blutdurchströmtes Blutgefäß etc.

Das Videoendoskop 20 weist an einem Griff 28 einen Endoskopschaft 22 auf, an dessen distalen Ende hinter einer Eintrittslinse 24 und einer nicht dargestellten Optik ein CMOS-Bildsensor 26 angeordnet ist. Das Videoendoskop 20 kann alternativ auch als Kombination eines herkömmlichen Endoskops mit einem Kamerakopf ausgebildet sein.

Das Videoendoskop 20 ist einerseits mit einer Lichtquelleneinheit 30 verbunden, die auch Bestandteil des Videoendoskops 20 sein kann oder, alternativ, Bestandteil einer Steuereinrichtung des endoskopischen Bildgebungssystems 10. Die Lichtquelleneinheit 30 ist mit verschiedenen Lichtquellen und Filtereinheiten ausgebildet, alternativ Weißlicht und Speziallicht gemäß einer oder mehrerer Speziallicht-Beleuchtungen zu erzeugen, welches durch das Videoendoskop 20 hindurch in die Körperhöhle 4 geleitet wird und das Untersuchungsumfeld ausgeleuchtet.

Das Bildgebungssystem 10 umfasst weiterhin eine Bildauswertungseinheit 40 und eine Steuereinheit 50, die ebenfalls mit der Lichtquelleneinheit 30 und dem Videoendoskop 20 verbunden sind, sowie eine Anzeigevorrichtung 60, die mit der Bildauswertungseinheit 40 verbunden ist. Die verschiedenen Komponenten, insbesondere die Bildauswertungseinheit 40 und die Steuereinheit 50, sind ausgebildet, das erfindungsgemäße Verfahren durchzuführen. Hierfür sind sie mit Programmcodemitteln bestückt, die die Bildauswertung und die Steuerung übernehmen.

In Fig. 2 ist das Bildgebungskonzept stigmatisiert dargestellt. Die Bildgebung und Bilderfassung beruhen auf der Erfassung von Weißlichtbildern 42, die einer Auswertung durch die Bildauswertungseinheit 40 unterzogen werden. Ein solches Weißlichtbild 42 kann beispielsweise eine HD-Auflösung aufweisen und wird unter Weißlichtbeleuchtung aus der Lichtquelleneinheit 30 erzeugt. Wenn das Weißlichtbild 42 eine Struktur 6 mit vordefinierter Charakteristik aufweist, beispielsweise die Charakteristik eines blutdurchströmten Gefäßes, einer Blutung, eines Darmpolypen etc., so ermittelt die Bildauswertungseinheit 40 den Teilbereich 44 des Weißlichtbildes 42, welcher die Struktur 6 beinhaltet.

Da eine Struktur 6 erkannt wurde, wird für das folgende Bild eine Speziallichtbeleuchtung für eine Speziallicht-Bildgebung eingestellt und der CMOS-Sensor 26 nur in dem Teilbereich 44 ausgelesen. Eine Darstellung, insbesondere Falschfarben-Darstellung, des ausgelesenen Teilbereichs 44 wird dem Weißlichtbild 42 an der richtigen Position als Speziallichtbild 46 überlagert, wodurch ein Kompositbild 48 erzeugt wird, das dem behandelnden Arzt auf der Anzeigevorrichtung 60 angezeigt wird. Das Kompositbild 48 kann Teil eines Videostreams im Speziallicht-Bildgebungsmodus sein, bei dem jeweils Paare von Weißlichtbildern und Speziallichtbildern aufgenommen und zusammengesetzt werden, oder als Einzelbild zur näheren Untersuchung und Dokumentation verwendet werden. Im Falle eines Videostreams im Speziallicht-Bildgebungsmodus wird vorzugsweise jedes Weißlichtbild analysiert und der Teilbereich 44 neu bestimmt, da sich das Videoendoskop 20 und die Struktur 6 zueinander relativ bewegen können.

Die Auswahl des konkreten Speziallicht-Bildgebungsmodus erfolgt entweder a priori durch den Arzt, der beispielsweise eine bestimmte Untersuchung, beispielsweise eine Darmspiegelung, durchführt, bei der nur ein Speziallicht-Bildgebungsmodus relevant ist, oder ein Bildauswertungsalgorithmus in der Bildauswertungseinheit 40 entscheidet aufgrund der gefundenen Struktur 6, welcher Speziallicht-Bildgebungsmodus ausgewählt wird. Ein solcher Auswahlalgorithmus kann beispielsweise auf neuronalen Netzen beruhen, die anhand von zuvor aufgenommenen Weißlichtbildern mit den entsprechenden Strukturen zur Erkennung und Klassifizierung der entsprechende Strukturen und der dafür optimalen Speziallicht-Bildgebungsmodi trainiert worden sind.

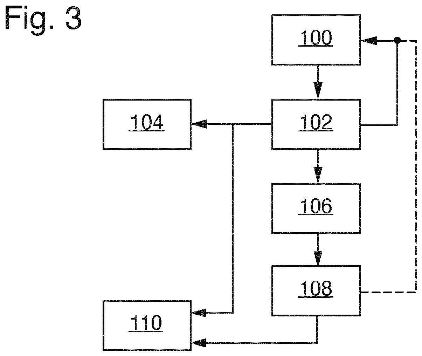

Fig. 3 zeigt ein beispielhaftes Ablaufdiagramm für ein erfindungsgemäßes Verfahren. Dieses beginnt mit der Aufnahme eines Weißlichtbildes 42 (Verfahrensschritt 100), welches nachfolgend auf das Vorhandensein einer Struktur 6 mit vorbestimmter Charakteristik hin analysiert wird (Verfahrensschritt 102). Falls keine Struktur 6 gefunden wird, wird das Weißlichtbild 42 als solches angezeigt (Verfahrensschritt 104) und ein neues Weißlichtbild 42 aufgenommen (Verfahrensschritt 100). Falls doch eine Struktur 6 in dem Weißlichtbild 42 gefunden wird, wird der Teilbereich 44 bestimmt, in dem sich die Struktur 6 im Weißlichtbild 42 befindet. Anschließend wird ein voreingestellter oder passender Speziallicht-Bildgebungsmodus mit einer entsprechenden Speziallicht-Beleuchtung eingestellt (Verfahrensschritt 106), eine Aufnahme unter der eingestellten Speziallicht-Beleuchtung gemacht und die Bilddaten des CMOS-Bildsensors 26 nur im Teilbereich 44 ausgelesen und als Speziallichtbild 46 weiterverarbeitet (Verfahrensschritt 108). Das Speziallichtbild 46 wird anschließend dem Weißlichtbild 42 überlagert und als Kompositbild 48 angezeigt (Verfahrensschritt 110). Der Vorgang wiederholt sich ab Verfahrensschritt 100, bis die Untersuchung beendet ist bzw. der erfindungsgemäße Untersuchungsmodus beendet wird.

### Bezugszeichenliste

- 2: Körper
- 4: Körperhohlraum
- 6: Struktur
- 10: endoskopisches Bildgebungssystem
- 20: Videoendoskop
- 22: Endoskopschaft
- 24: Eintrittslinse
- 26: CMOS-Bildsensor
- 28: Griff
- 30: Lichtquelleneinheit
- 40: Bildauswertungseinheit
- 42: Weißlichtbild
- 44: Teilbereich
- 46: Speziallichtbild
- 48: Kompositbild
- 50: Steuereinheit
- 60: Anzeigevorrichtung
- 100: Aufnahme eines Weißlichtbildes
- 102: Analyse des Weißlichtbildes
- 104: Anzeige des Weißlichtbildes
- 106: Einstellen eines Speziallicht-Bildgebungsmodus
- 108: Aufnahme eines Speziallichtbildes
- 110: Erzeugen und Anzeigen eines Kompositbildes

## Patentansprüche

1. Verfahren zur endoskopischen Bildgebung mit einem endoskopischen Bildgebungssystem (10), das ein Videoendoskop (20) mit wenigstens einem CMOS-Bildsensor (26), eine Lichtquelleneinheit (30), die zur Erzeugung von Weißlicht sowie von wenigstens einem Speziallicht ausgebildet ist, eine Bildauswertungseinheit (40), eine Steuereinheit (50) und eine Anzeigevorrichtung (60) umfasst,
**dadurch gekennzeichnet,**
**dass** mit dem Videoendoskop (20) unter Weißlichtbeleuchtung Weißlichtbilder (42) aufgenommen und von der Bildauswertungseinheit (40) in Echtzeit auf das Vorhandensein wenigstens einer Struktur (6) mit wenigstens einer vordefinierten Charakteristik hin ausgewertet werden,
wobei, wenn die Bildauswertungseinheit (40) in wenigstens einem Weißlichtbild (42) das Vorhandensein wenigstens einer Struktur (6) mit wenigstens einer vordefinierten Charakteristik erkennt, ein Speziallicht-Bildgebungsmodus eingestellt wird, in welchem die Lichtquelleneinheit (30) eine Speziallichtbeleuchtung mit dem wenigstens einen Speziallicht erzeugt und eine oder mehrere Bildaufnahmen eines Videostreams unter der Speziallichtbeleuchtung aufgenommen wird oder werden, die einer Bildverarbeitung in einem Speziallichtverarbeitungsmodus unterzogen wird oder werden, welche umfasst, dass ein Teilbereich (44) des Weißlichtbildes (42) identifiziert wird, der die im Weißlichtbild erkannte Struktur (6) mit vordefinierter Charakteristik enthält und nur dieser Teilbereich (44) des Bildsensors (26) ausgelesen wird, wobei die aus dem Teilbereich (44) ausgelesenen Bilddaten als Speziallichtbild (46) oder Speziallichtbilder (46) weiterverarbeitet werden.

2. Verfahren nach Anspruch 1, **dadurch gekennzeichnet, dass** die Weißlichtbilder (42) mittels der Anzeigevorrichtung (60) angezeigt werden, wenn die Bildauswertungseinheit (40) in den Weißlichtbildern (42) keine Struktur mit wenigstens einer vordefinierten Charakteristik erkennt.

3. Verfahren nach Anspruch 1 oder 2, **dadurch gekennzeichnet, dass** das Speziallichtbild (46) oder die Speziallichtbilder (46) alleine oder als Kompositbild (48) oder Kompositbilder (48) in einer Überlagerung über Weißlichtbilder (42) mittels der Anzeigevorrichtung (60) angezeigt wird oder werden.

4. Verfahren nach einem der Ansprüche 1 bis 3, **dadurch gekennzeichnet, dass** in dem Fall, wenn die Bildauswertungseinheit (40) in den Weißlichtbildern (42) das Vorhandensein wenigstens einer Struktur (6) mit wenigstens einer vordefinierten Charakteristik erkennt, eine mit der Video-Bildfolge synchronisierte alternierende Weißlicht- und Speziallichtbeleuchtung erzeugt wird, wobei jeweils ein Paar aus Weißlichtbild (42) und Speziallichtbild (46) verarbeitet und zu einem Kompositbild (48) zusammengesetzt wird.

5. Verfahren nach Anspruch 4, **dadurch gekennzeichnet, dass** die Video-Bildfolge und die synchronisierte alternierende Weißlicht- und Speziallichtbeleuchtung in einem zeitlichen Rhythmus erfolgt, in dem die Dauer der Speziallichtbeleuchtung und Speziallichtbildaufnahme kürzer ist als die Dauer der Weißlichtbeleuchtung und Weißlichtbildaufnahme.

6. Verfahren nach einem der Ansprüche 1 bis 5, **dadurch gekennzeichnet, dass** die Lichtquelleneinheit (30) ausgebildet ist, eine oder mehrere aus einer Beleuchtung für Schmalband-Bildgebung (NBI), einer Beleuchtung für Nahinfrarotfluoreszenz-Bildgebung (NIRF) und einer Beleuchtung für eine Rot-Dichromatische-Bildgebung (RDI) als Speziallicht zu erzeugen, wobei insbesondere bei erkannter Blutung eine RDI-Beleuchtung erzeugt wird und/oder bei erkannten Darmpolypen eine NBI-Beleuchtung erzeugt wird.

7. Verfahren nach einem der Ansprüche 1 bis 6, **dadurch gekennzeichnet, dass** die Bildauswertungsvorrichtung (40) anhand der erkannten Struktur (6) mit vordefinierter Charakteristik eine aus mehreren zur Verfügung stehenden Speziallicht-Modi auswählt.

8. Verfahren nach einem der Ansprüche 1 bis 7, **dadurch gekennzeichnet, dass** die vordefinierte Charakteristik und der Speziallicht-Modus vorausgewählt und eingestellt ist oder wird.

9. Verfahren nach einem der Ansprüche 1 bis 8, **dadurch gekennzeichnet, dass** die Erkennung der Struktur (6) mit wenigstens einer vordefinierten Charakteristik und/oder die Auswahl des die Struktur (6) enthaltenden Teilbereichs (44) und/oder die Auswahl des Speziallicht-Modus mittels eines auf künstlicher Intelligenz basierenden Bildauswertungsalgorithmus erfolgt.

10. Verfahren nach Anspruch 9, **dadurch gekennzeichnet, dass** der Bildauswertungsalgorithmus auf einem oder mehreren klassifizierenden Neuronalen Netzen, insbesondere CNN, beruht, welches oder welche mit Weißlichtbildern (42) vortrainiert wurde oder wurden, die Strukturen (6) mit wenigstens einer vordefinierten Charakteristik enthalten.

11. Verfahren nach einem der Ansprüche 1 bis 9, **dadurch gekennzeichnet, dass** die Bildauswertungseinheit (40) die Speziallichtbilder (46) auf das Vorhandensein einer entsprechenden Struktur (6) hin auswertet.

12. Endoskopisches Bildgebungssystem (10), umfassend ein Videoendoskop (20) mit wenigstens einem CMOS-Bildsensor (26), eine Lichtquelleneinheit (30), die zur Erzeugung von Weißlicht sowie von wenigstens einem Speziallicht ausgebildet ist, eine Bildauswertungseinheit (40), eine Steuereinheit (50) und eine Anzeigevorrichtung (60), wobei die Steuereinheit (50) mit der Lichtquelleneinheit (30), dem Videoendoskop (20) und der Bildauswertungseinheit (40) signaltechnisch verbunden ist und die Bildauswertungseinheit (40) mit dem Videoendoskop (20) signaltechnisch verbunden ist, **dadurch gekennzeichnet, dass** das Bildgebungssystem (10) mit dem Videoendoskop (20), der Lichtquelleneinheit (30), der Bildauswertungseinheit (40), der Steuereinheit (50) und der Anzeigevorrichtung (60) ausgebildet und eingerichtet ist, ein Verfahren nach einem der Ansprüche 1 bis 11 auszuführen.

13. Softwareprogrammprodukt mit Programmcodemitteln für ein endoskopisches Bildgebungssystem (10) nach Anspruch 12, mit einer Bildauswertungsprogrammkomponente, die in der Bildauswertungseinheit (40) des Bildgebungssystems (10) abläuft, und einer Steuerprogrammkomponente, die in der Steuereinheit (50) des Bildgebungssystems (10) abläuft, **dadurch gekennzeichnet, dass** die Bildauswertungsprogrammkomponente und die Steuerprogrammkomponente ausgebildet sind, ein Verfahren nach einem der Ansprüche 1 bis 11 auszuführen, wenn sie in der Bildauswertungseinheit (40) und der Steuereinheit (50) ablaufen.

## Claims

1. A method for endoscopic imaging with an endoscopic imaging system (10) which comprises a video endoscope (20) having at least one CMOS image sensor (26), a light source unit (30) designed to generate white light and at least one special light, an image evaluation unit (40), a control unit (50) and a display device (60),
**characterized in that**
white light images (42) are captured by means of the video endoscope (20) under white light illumination and evaluated by the image evaluation unit (40) in real time to check for the presence of at least one structure (6) having at least one predefined characteristic,
wherein, when the image evaluation unit (40) detects the presence of at least one structure (6) having at least one predefined characteristic in at least one white light image (42), a special light imaging mode is set in which the light source unit (30) generates special light illumination using the at least one special light and one or more images of a video stream is or are captured under the special light illumination and is or are subjected to image processing in a special light processing mode that comprises identifying a subregion (44) of the white light image (42) that contains the structure (6) with the predefined characteristic detected in the white light image and reading out only this subregion (44) of the image sensor (26), wherein the image data read out from the subregion (44) are further processed as a special light image (46) or special light images (46).

2. The method according to claim 1, **characterized in that** the white light images (42) are displayed by means of the display device (60) if the image evaluation unit (40) detects no structure having at least one predefined characteristic in the white light images (42).

3. The method according to claim 1 or 2, **characterized in that** the special light image (46) or special light images (46) is or are displayed alone or as a composite image (48) or as composite images (48) superimposed on white light images (42) by means of the display device (60).

4. The method according to any one of claims 1 to 3, **characterized in that**, in cases where the image evaluation unit (40) detects the presence of at least one structure (6) having at least one predefined characteristic in the white light images (42), alternating white light and special light illumination is generated synchronized with the video image sequence, wherein in each case one pair consisting of a white light image (42) and a special light image (46) is processed and combined into a composite image (48).

5. The method according to claim 4, **characterized in that** the video image sequence and the synchronized alternating white light and special light illumination occurs in a temporal rhythm in which the duration of the special light illumination and special light image capture is shorter than the duration of the white light illumination and white light image capture.

6. The method according to any one of claims 1 to 5, **characterized in that** the light source unit (30) is designed to generate one or more of the following as special light: illumination for narrow band imaging (NBI), illumination for near-infrared fluorescence imaging (NIRF) and illumination for red dichromatic imaging (RDI), wherein RDI illumination is generated in particular in the case of detected bleeding and/or NBI illumination is generated in the case of detected intestinal polyps.

7. The method according to any one of claims 1 to 6, **characterized in that** the image evaluation device (40) is designed to select one of multiple special light modes available based on the detected structure (6) having the predefined characteristic.

8. The method according to any one of claims 1 to 7, **characterized in that** the predefined characteristic and the special light mode are or have been preselected and set.

9. The method according to any one of claims 1 to 8, **characterized in that** the structure (6) having at least one predefined characteristic is detected and/or the subregion (44) containing the structure (6) is selected and/or the special light mode is selected by means of an image evaluation algorithm based on artificial intelligence.

10. The method according to claim 9, **characterized in that** the image evaluation algorithm is based on one or more classifying neural networks, in particular CNN, which has or have been pretrained using white light images (42) containing the structures (6) having at least one predefined characteristic.

11. The method according to any one of claims 1 to 9, **characterized in that** the image evaluation unit (40) evaluates the special light images (46) to check for the presence of a corresponding structure (6).

12. An endoscopic imaging system (10), comprising a video endoscope (20) having at least one CMOS image sensor (26), a light source unit (30) designed to generate white light and at least one special light, an image evaluation unit (40), a control unit (50) and a display device (60), wherein the control unit (50) is connected to the light source unit (30), video endoscope (20) and image evaluation unit (40) for signaling and the image evaluation unit (40) is connected to the video endoscope (20) for signaling, **characterized in that** the imaging system (10) having the video endoscope (20), light source unit (30), image evaluation unit (40), control unit (50) and display device (60) is designed and configured to carry out a method according to any one of claims 1 to 11.

13. A software program product comprising program code means for an endoscopic imaging system (10) according to claim 12, comprising an image evaluation program component that is executed in the image evaluation unit (40) of the imaging system (10), and a control program component that is executed in the control unit (50) of the imaging system (10), **characterized in that** the image evaluation program component and the control program component are designed to carry out a method according to any one of claims 1 to 11 when they are executed in the image evaluation unit (40) and control unit (50).

## Revendications

1. Procédé d'imagerie endoscopique avec un système d'imagerie endoscopique (10) qui comprend un endoscope vidéo (20) avec au moins un capteur d'image CMOS (26), une unité (30) formant source de lumière qui est conçue pour produire de la lumière blanche ainsi qu'au moins une lumière spéciale, une unité (40) d'évaluation d'images, une unité de commande (50) et un dispositif d'affichage (60),
**caractérisé en ce que**
des images (42) sous lumière blanche sont prises avec l'endoscope vidéo (20) sous un éclairage de lumière blanche et sont évaluées en temps réel par l'unité (40) d'évaluation d'images en ce qui concerne la présence d'au moins une structure (6) ayant au moins une caractéristique prédéfinie ;
lorsque l'unité (40) d'évaluation d'images reconnaît dans au moins une image (42) sous lumière blanche la présence d'au moins une structure (6) ayant au moins une caractéristique prédéfinie, un mode d'imagerie de lumière spéciale est réglé, dans lequel l'unité (30) formant source de lumière génère un éclairage de lumière spéciale par ladite au moins une lumière spéciale et une ou plusieurs prises de vue d'un flux vidéo sont enregistrées sous l'éclairage de lumière spéciale, lesquelles sont soumises à un traitement d'image dans un mode de traitement de lumière spéciale qui comprend l'identification d'une zone partielle (44) de l'image (42) sous lumière blanche qui contient la structure (6) reconnue dans l'image de lumière blanche avec une caractéristique prédéfinie et la lecture uniquement de cette zone partielle (44) du capteur d'image (26), les données d'image lues à partir de la zone partielle (44) étant traitées ultérieurement en tant qu'image de lumière spéciale (46) ou images de lumière spéciale (46).

2. Procédé selon la revendication 1, **caractérisé en ce que** les images (42) sous lumière blanche sont affichées au moyen du dispositif d'affichage (60) lorsque l'unité (40) d'évaluation d'images ne reconnaît pas de structure présentant au moins une caractéristique prédéfinie dans les images (42) sous lumière blanche.

3. Procédé selon la revendication 1 ou la revendication 2, **caractérisé en ce que** l'image lumineuse spéciale (46) ou les images lumineuses spéciales (46) est ou sont affichées seules ou sous forme d'image composite (48) ou d'images composites (48) en superposition sur des images (42) sous lumière blanche au moyen du dispositif d'affichage (60).

4. Procédé selon l'une des revendications 1 à 3, **caractérisé en ce que** dans le cas où l'unité (40) d'évaluation d'images détecte dans les images (42) sous lumière blanche la présence d'au moins une structure (6) ayant au moins une caractéristique prédéfinie, il est généré un éclairage alterné en lumière blanche et en lumière spéciale synchronisé avec la séquence d'images vidéo, une paire d'image (42) sous lumière blanche et d'image (46) sous lumière spéciale étant traitée et assemblée pour former une image composite (48).

5. Procédé selon la revendication 4, **caractérisé en ce que** la séquence d'images vidéo et l'alternance synchronisée de l'éclairage en lumière blanche et de l'éclairage spécial s'effectuent à un rythme temporel dans lequel la durée de l'éclairage spécial et de la prise de vue en lumière spéciale est inférieure à la durée de l'éclairage en lumière blanche et de la prise de vue en lumière blanche.

6. Procédé selon l'une des revendications 1 à 5, **caractérisé en ce que** l'unité (30) formant source de lumière est conçue pour produire un ou plusieurs éclairages parmi un éclairage pour l'imagerie à bande étroite (NBI), un éclairage pour l'imagerie par fluorescence dans le proche infrarouge (NIRF) et un éclairage pour une imagerie dichromatique rouge (RDI) en tant que lumière spéciale, un éclairage RDI étant notamment produit en cas de détection d'une hémorragie et/ou un éclairage NBI étant produit en cas de détection de polypes intestinaux.

7. Procédé selon l'une des revendications 1 à 6, **caractérisé en ce que** l'unité (40) d'évaluation d'images sélectionne, à l'aide de la structure (6) détectée ayant une caractéristique prédéfinie, un mode d'éclairage spécial parmi plusieurs modes disponibles.

8. Procédé selon l'une des revendications 1 à 7, **caractérisé en ce que** la caractéristique prédéfinie et le mode d'éclairage spécial sont présélectionnés et réglés.

9. Procédé selon l'une des revendications 1 à 8, **caractérisé en ce que** la détection de la structure (6) ayant au moins une caractéristique prédéfinie et/ou la sélection de la zone partielle (44) contenant la structure (6) et/ou la sélection du mode d'éclairage spécial est effectuée au moyen d'un algorithme d'évaluation d'image basé sur l'intelligence artificielle.

10. Procédé selon la revendication 9, **caractérisé en ce que** l'algorithme d'évaluation d'image est basé sur un ou plusieurs réseaux neuronaux classificateurs, notamment CNN, qui a ou ont été pré-entraînés avec des images (42) sous lumière blanche contenant des structures (6) avec au moins une caractéristique prédéfinie.

11. Procédé selon l'une des revendications 1 à 9, **caractérisé en ce que** l'unité (40) d'évaluation d'images évalue les images (46) sous lumière spéciale en fonction de la présence d'une structure (6) correspondante.

12. Système d'imagerie endoscopique (10), comprenant un endoscope vidéo (20) ayant au moins un capteur d'image CMOS (26), une unité (30) formant source de lumière qui est conçue pour générer de la lumière blanche ainsi qu'au moins une lumière spéciale, une unité (40) d'évaluation d'images, une unité de commande (50) et un dispositif d'affichage (60), l'unité de commande (50) étant reliée à l'unité (30) formant source de lumière, à l'endoscope vidéo (20) et à l'unité (40) d'évaluation d'images, et l'unité (40) d'évaluation d'images est reliée à l'endoscope vidéo (20) selon une technique de signalisation, **caractérisé en ce que** le système d'imagerie (10), avec l'endoscope vidéo (20), l'unité (30) formant source de lumière, l'unité (40) d'évaluation d'images, l'unité de commande (50) et le dispositif d'affichage (60), est conçu et agencé pour mettre en œuvre un procédé selon l'une des revendications 1 à 11.

13. Produit de programme d'ordinateur avec des moyens de code de programme pour un système d'imagerie endoscopique (10) selon la revendication 12, ayant un composant de programme d'évaluation d'image qui s'exécute dans l'unité (40) d'évaluation d'images du système d'imagerie (10), et un composant de programme de commande, qui s'exécute dans l'unité de commande (50) du système d'imagerie (10), **caractérisé en ce que** le composant de programme d'évaluation d'image et le composant de programme de commande sont conçus pour mettre en œuvre un procédé selon l'une des revendications 1 à 11 lorsqu'ils s'exécutent dans l'unité (40) d'évaluation d'images et l'unité de commande (50).
